(19) **Europäisches Patentamt / European Patent Office / Office européen des brevets**

(11) **EP 4 000 511 A1**

(12) # EUROPEAN PATENT APPLICATION

(43) Date of publication:
**25.05.2022 Bulletin 2022/21**

(21) Application number: **20207161.9**

(22) Date of filing: **12.11.2020**

(51) International Patent Classification (IPC):
**A61B 5/055** (2006.01)    **A61B 5/16** (2006.01)
**G01R 33/48** (2006.01)

(52) Cooperative Patent Classification (CPC):
**A61B 5/165; A61B 5/055;** A61B 5/162; A61B 5/163;
G01R 33/4806

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
 • VALEV, Hristo Ventzeslavov
  **5656 AE Eindhoven (NL)**

 • LEUFKENS, Timmy Robertus Maria
  **5656 AE Eindhoven (NL)**
 • WESTERINK, Joanne Henriëtte Desirée Monique
  **5656 AE Eindhoven (NL)**
 • VAN DOOREN, Marieke
  **5656 AE Eindhoven (NL)**
 • VAN EE, Raymond
  **5656 AE Eindhoven (NL)**
 • HUIJBERS, Willem
  **5656 AE Eindhoven (NL)**

(74) Representative: **Philips Intellectual Property & Standards**
**High Tech Campus 52**
**5656 AG Eindhoven (NL)**

(54) **APPARATUS FOR DETERMINING AND/OR ASSESS DEPRESSION SEVERITY OF A PATIENT**

(57)    The present invention relates to an apparatus (10) for determining and/or assessing depression severity of a patient. The apparatus comprising: an input unit (20); an image display unit (30); at least one sensor (40); and a processing unit (50). The image display unit is configured to present a plurality of images to a patient and the plurality of images are of different human facial expressions. The at least one sensor is configured to acquire information relating to the patient's viewing of the plurality of images. The input unit is configured to provide the processing unit with the information relating to the patient's viewing of the plurality of images. The input unit is configured to provide the processing unit with MRI scan image data of the patient. The MRI scan image data of the patient comprises MRI scan image data of at least one part of the patient's brain associated with recognition of facial expressions and/or associated with affective disorders. The processing unit is configured to determine and/or assess depression severity of the patient comprising utilization of the information relating to the patient's viewing of the plurality of images and the MRI scan image data of at least one part of the patient's brain associated with recognition of facial expressions

FIG. 1

EP 4 000 511 A1

**Description**

FIELD OF THE INVENTION

The present invention relates to an apparatus for determining and/or assessing depression severity of a patient, an imaging system, a method for determining and/or assessing depression severity of a patient, as well as to a computer program element and a computer readable medium.

BACKGROUND OF THE INVENTION

Determining and/or assessing depression severity of a patient is typically carried out via an assessment of mood disorders of the patient. Diagnosis of mood disorders is traditionally done via a psychiatric assessment of symptoms through questionnaires. A typical questionnaire retrospectively probes psychiatric symptoms from the previous weeks. The main disadvantage of questionnaires is that they are error prone due to failed memory recall, retrospective biases and socially acceptable answers. Furthermore, questionnaires are also prone to communication errors between patient and examiner (e.g. psychiatrist, psychologist). Communication errors can be aggravated in patients with deficits in verbal or written communication. Another shortcoming of subjective questionnaires is that they lose accuracy if used frequently.
There is a need to address these issues.

SUMMARY OF THE INVENTION

It would be advantageous to have improved means of determining and/or assessing depression severity of a patient. The object of the present invention is solved with the subject matter of the independent claims, wherein further embodiments are incorporated in the dependent claims. It should be noted that the following described aspects and examples of the invention apply also to the apparatus for determining and/or assessing depression severity of a patient, the imaging system, and method for determining and/or assessing depression severity of a patient, as well as to a computer program element and a computer readable medium.
In a first aspect, there is provided an apparatus for determining and/or assessing depression severity of a patient, the apparatus comprising:

- an input unit;
- an image display unit;
- at least one sensor; and
- a processing unit.

The image display unit is configured to present a plurality of images to a patient and the plurality of images are of different human facial expressions. The at least one sensor is configured to acquire information relating to the patient's viewing of the plurality of images. The input unit is configured to provide the processing unit with the information relating to the patient's viewing of the plurality of images. The input unit is configured to provide the processing unit with Magnetic Resonance Imaging (MRI) scan image data of the patient. The MRI scan image data of the patient comprises MRI scan image data of at least one part of the patient's brain associated with recognition of facial expressions and/or associated with affective disorders. The processing unit is configured to determine and/or assess depression severity of the patient comprising utilization of the information relating to the patient's viewing of the plurality of images and the MRI scan image data of at least one part of the patient's brain associated with recognition of facial expressions and/or associated with affective disorders.
In other words, a patient views a number of facial expressions, for example ranging from sad to happy and that can progressively range from most sad to most happy and information relating to this viewing is acquired, and at the same time and/or at a different time that this information was acquired the patient views these facial expressions, and MRI scan images, for example functional MRI scan images of the parts of the brain is associated with processing facial expressions and/or associated with affective disorders is acquired. Then, the information relating to how the patient has viewed these facial expressions (whether during the MRI scan and/or at a different time) is analysed along with the MRI scan images acquired as the patient views these images from which the determination and/or assessment of a degree of depression of the patient can be made.
In an example, at least some of the MRI scan image data of the patient comprises MRI scan image data of the patient acquired during a period when the plurality of images were presented to the patient.
In an example, at least some of the information relating to the patient's viewing of the plurality of images was acquired at the same time as the MRI scan image data of at least one part of the patient's brain associated with recognition of facial expressions and/or associated with affective disorders.

In an example, the image display unit comprises an interaction input unit configured to present different images to the patient in response to input provided by the patient. A sensor of the at least one sensor is configured to determine what image of the plurality of images the patient is viewing. The information relating to the patient's viewing of the plurality of images relates to the viewing of one or more images of the plurality of images.

In other words, the patient is viewing images of human faces ranging from sad to happy, and the patient themselves can control via a slider or pushing forward or backward buttons or using a touchpad or even using a mouse on the image display unit which faces the view. The way the patient controls and views these images can be analysed along with the MRI data acquired at the same time and/or at a different time from which the degree of depression can be determined and/or assessed.

In an example, the information relating to the patient's viewing of the plurality of images comprises information relating to at least one image browsing pattern of the patient.

In this manner, how the patient interacts with the image display unit in presenting images to themselves, for example how quickly they progress from sad images to happy images, and whether and how many times they backtrack in terms of not continuously going from sad to happy, but reverting from happy to a slightly sadder and then maybe going back in the happy direction and how long the patient dwells in different images can all be used along with the MRI data required at the same time in determining and/or assessing depression severity of the patient.

In an example, the information relating to the at least one browsing pattern comprises of one more of: durations that the patient has viewed two or more images of the plurality of images; changes in input by the patient to the interaction input unit with respect to changes in a viewing direction of the plurality of images.

In an example, a sensor of the at least one sensor comprises an eye tracking sensor. The information relating to the patient's viewing of the plurality of images can then comprise information relating to where the patient is looking.

In this manner, at the same time that MRI data is acquired of the patient looking at a plurality of images, the exact position of where the patient is actually viewing those images can be acquired and this information used with the MRI data enables a depression state and severity of the patient to be determined and/or assessed. Also, if the images are viewed at a different time to the MRI scan, the information of exactly where the patient is viewing can be used to help inform for example where to subsequently MRI image the brain and/or otherwise be used along with the MRI data in determining the depressive severity of the patient.

In an example, the processing unit is configured to utilize the information relating to the patient's viewing of the plurality of images and the MRI scan image data of at least one part of the patient's brain associated with recognition of facial expressions and/or associated with affective disorders to determine a distribution of assessments of facial expressions for the plurality of images and wherein the determination and/or assessment of the depression severity of the patient comprises a determination of a skewness metric associated with the distribution of assessments of facial expressions with respect to that exhibited by one or more non-depressed patients.

In an example, the image display unit is configured to present a first image of the plurality of images to the left visual field of the patient and present a second image of the plurality of images to the right visual field of the patient. The processing unit is configured to determine which image of the first image or second image is gaining the most attention from the patient. The information relating to the patient's viewing of the plurality of images can comprise information relating to the image that is gaining the most attention by the patient.

Thus, the patient is provided with one image to the left visual field, and provided with another image for the right visual field that is of a happier disposition than for the left visual field. At the same time MRI data is acquired of those parts of the brain's associated with processing information in the left visual field and right visual field, from which it can be determined which of these two images is the image that the patient is concentrating on and this along with the MRI data from this past the brain's associated recognition of facial expressions enables the depressive severity of the patient to determine that recessed.

In an example, the MRI image data of the patient comprises image data of at least one part of the brain associated with viewing an image with the right visual field and image data of at least one part of the brain associated with viewing an image with the left visual field. The processing unit is configured to analyse the image data of the at least one part of the brain associated with viewing an image with the right visual field and the image data of at least one part of the brain associated with viewing an image with the left visual field to determine which image is gaining the most attention by the patient.

In an example, an image of the plurality of images is an adapted image. The processing unit is configured to adapt a facial expression of an image to provide the adapted image comprising utilization of the information relating to the patient's viewing of one or more images of the plurality of images.

In other words, an adaptive feedback systems provided, in which as the patient scans through images and MRI data is acquired as well as data relating to how the patient is viewing the images, can be used to adapt an image that the patient has yet to see in order that the most appropriate images can be provided to the patient thereby increasing the accuracy of the assessment of the depressive severity.

In a second aspect, there is provided an imaging system comprising;

- a magnetic resonance imaging (MRI) scanner; and
- an apparatus for determining and/or assessing depression severity of a patient according to the first aspect.

In a third aspect, there is provided a method for determining and/or assessing depression severity of a patient, the method comprising:

a) presenting on an image display unit a plurality of images to a patient, and wherein the plurality of images are of different human facial expressions;

b) acquiring by at least one sensor information relating to the patient's viewing of the plurality of images;

c) providing by an input unit a processing unit with the information relating to the patient's viewing of the plurality of images;

d) providing by the input unit the processing unit with MRI scan image data of the patient, wherein the MRI scan image data of the patient comprises MRI scan image data of at least one part of the patient's brain associated with recognition of facial expressions and/or associated with affective disorders; and

e) determining and/or assessing by the processing unit depression severity of the patient comprising utilizing the information relating to the patient's viewing of the plurality of images and the MRI image data of at least one part of the patient's brain associated with recognition of facial expressions and/or associated with affective disorders.

According to another aspect, there is provided a computer program element controlling one or more of the apparatuses or system as previously described which, if the computer program element is executed by a processing unit, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

BRIEF DESCRIPTION OF THE DRAWINGS

Exemplary embodiments will be described in the following with reference to the following drawing:

Fig. 1 shows a schematic set up of an example of an apparatus for determining and/or assessing depression severity of a patient;

Fig. 2 shows a schematic set up of an example of an imaging system;

Fig. 3 shows a method for determining and/or assessing depression severity of a patient; and

Fig. 4 shows schematically how the visual system is retinotopically organized.

DETAILED DESCRIPTION OF EMBODIMENTS

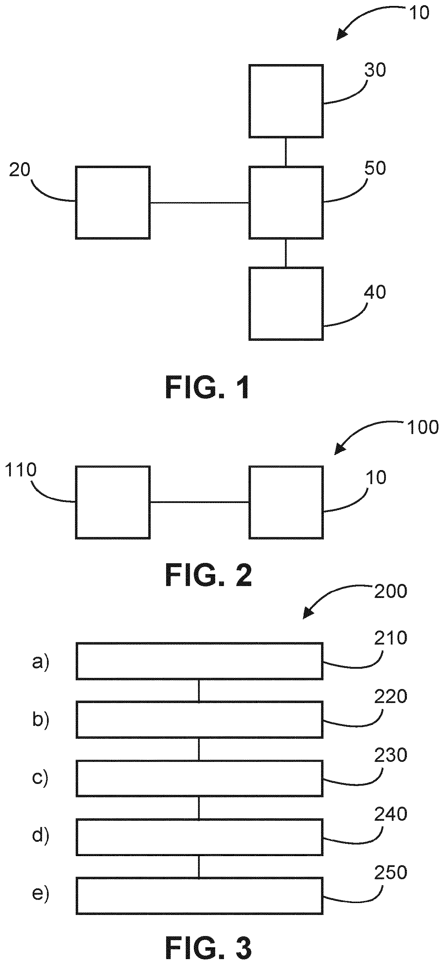

Fig. 1 shows a schematic example of an apparatus 10 for determining and/or assessing depression severity of a patient. The apparatus comprises an input unit 20, an image display unit 30, at least one sensor 40, and a processing unit 50. The image display unit is configured to present a plurality of images to a patient and the plurality of images are of different human facial expressions. The at least one sensor is configured to acquire information relating to the patient's viewing of the plurality of images. The input unit is configured to provide the processing unit with the information relating to the patient's viewing of the plurality of images. The input unit is configured to provide the processing unit with MRI scan image data of the patient. The MRI scan image data of the patient comprises MRI scan image data of at least one part of the patient's brain associated with recognition of facial expressions and/or associated with affective disorders. The processing unit is configured to determine and/or assess depression severity of the patient comprising utilization of the information relating to the patient's viewing of the plurality of images and the MRI scan image data of at least one part of the patient's brain associated with recognition of facial expressions and/or associated with affective disorders.

Thus, in a specific example a patient can viewing image in the days before the MRI scan, and that from his interaction with these images a prediction is made as to what type of depression he has, and consequently, which parts of the brain need to be imaged in the MRI scan. This means that the viewing of the images will provide guidance to the medical staff/neurologist as to which parts of the brain should be targeted in the MRI scan. Then the MRI scan data acquired can be analysed with respect to depression, and this analysis could itself also take into account how the patient viewed the images. Thus, the information relating to the image viewing and the information relating to the MRI data can be acquired at different times and processed at different times with respect to determining depression severity, but also include the information being processed together.

It is to be noted that reference to "input unit" could refer to more than one input unit. Thus, one input unit can provide the processing unit with the information relating to the patient's viewing of the plurality of images, and a separate input unit can provide the processing unit with MRI scan image data of the patient. Thus, input unit can refer to the means by which this information/data is presented to the processing unit, and could indeed be a single input unit.

It is to be noted that reference to "processing unit" could similarly refer to more than one processing unit. Thus, one processing unit can be provided with the information relating to the patient's viewing of the plurality of images and determine for example where it might be best to MRI image the patient's brain. Then a separate processing unit can be provided with MRI scan image data of the patient and process that, and could also be provided with the information derived from image viewing. However, a single processor can also carry out these processing functions. Thus, processing unit can refer to the means by which this information/data is processed, and could be via two separate processing units or be a single processing unit.

In an example, the image display unit is configured to present the plurality of images to the patient undergoing the MRI scan.

In an example, a sensor of the at least one sensor is configured to determine what image of the plurality of images the patient is viewing.

According to an example, at least some of the MRI scan image data of the patient comprises MRI scan image data of the patient acquired during a period when the plurality of images were presented to the patient.

According to an example, at least some of the information relating to the patient's viewing of the plurality of images was acquired at the same time as the MRI scan image data of at least one part of the patient's brain associated with recognition of facial expressions and/or associated with affective disorders.

In an example, at least some of the information relating to the patient's viewing of the plurality of images was acquired at a different time to the MRI scan image data of at least one part of the patient's brain associated with recognition of facial expressions and/or associated with affective disorders

According to an example, the image display unit comprises an interaction input unit configured to present different images to the patient in response to input provided by the patient. A sensor of the at least one sensor is configured to determine what image of the plurality of images the patient is viewing. The information relating to the patient's viewing of the plurality of images can then relate to the viewing of one or more images of the plurality of images.

In an example, the interaction input unit comprises a slidable scale input or push button or touchpad or mouse configured to enable the patient to browse through the plurality of images from one type of emotion or feeling or mood state to another, for example from sad to happy and from happy to sad.

Thus, a slidable scale or other input means (push buttons, touchpad, mouse etc.) enables a patient to browse through the images, and an associated sensor provides information on what image is being viewed - thus here sensor can be an output of a processing unit of the image display unit for example that details what images are being viewed and when and this sensor can also provide information regarding how the slidable scale (or other input means) is being used in viewing images.

In an example, the information relating to the patient's viewing of the plurality of images comprises information relating to an image that the patient views that the patient indicates to equate with a current state of the patient.

Thus, the patient is requested to determine an image themselves that correlates most with how they feel at that time, and this image and the associated MRI data required, and indeed how the patient has scan to other images in resulting at this image along with the associated MRI data can be utilised in determining and/or assessing the depressive state of the patient.

According to an example, the information relating to the patient's viewing of the plurality of images comprises information relating to at least one image browsing pattern of the patient.

According to an example, the information relating to the at least one browsing pattern comprises of one more of: durations that the patient has viewed two or more images of the plurality of images; changes in input by the patient to the interaction input unit with respect to changes in a viewing direction of the plurality of images.

In an example, the information relating to the patient's viewing of the plurality of images comprises information relating to a one or more specific images viewed by the patient.

Thus, this could be any image that the patient views or an average of multiple selections by the patient or another weighted sum of multiple observations of the patient who is viewing/interacting with the images. Thus, during an MRI scan and/or at a different time to the MRI scan the patient can repeat the interaction with the faces several times (with either different starting point, or different faces), in order that repeated measurement if required are obtained.

To put this another way, a patient can see the same image multiple times inside the MRI scanner and/or outside of the scanner at a different time to an MRI scan, and thus make repeated interactions with the same face/stimulus. Thus in effect, unique facial stimuli can be used during an MRI scan, and where post or (pre)scan interaction with the same stimuli can be used to inform the analysis and thus estimates of brain activity. Thus, a particular stimulus (image) may need only be shown to the patient once whilst undertaking an MRI scan, but the analysis of the brain scan is informed by multiple (aggregated) interactions with the same stimuli for example outside of the scanner that were acquired at different times to the MRI scan image data.

According to an example, a sensor of the at least one sensor comprises an eye tracking sensor. The information relating to the patient's viewing of the plurality of images can then comprise information relating to where the patient is looking.

According to an example, the processing unit is configured to utilize the information relating to the patient's viewing of the plurality of images and the MRI scan image data of at least one part of the patient's brain associated with recognition of facial expressions and/or associated with affective disorders to determine a distribution of assessments of facial expressions for the plurality of images and wherein the determination and/or assessment of the depression severity of the patient comprises a determination of a skewness metric associated with the distribution of assessments of facial expressions with respect to that exhibited by one or more non-depressed patients.

According to an example, the image display unit is configured to present a first image of the plurality of images to the left visual field of the patient and present a second image of the plurality of images to the right visual field of the patient. The processing unit is configured to determine which image of the first image or second image is gaining the most attention from the patient. The information relating to the patient's viewing of the plurality of images can then comprise information relating to the image that is gaining the most attention by the patient.

According to an example, the MRI image data of the patient comprises image data of at least one part of the brain associated with viewing an image with the right visual field and image data of at least one part of the brain associated with viewing an image with the left visual field. The processing unit is configured to analyse the image data of the at least one part of the brain associated with viewing an image with the right visual field and the image data of at least one part of the brain associated with viewing an image with the left visual field to determine which image is gaining the most attention by the patient.

According to an example, an image of the plurality of images is an adapted image. The processing unit is configured to adapt a facial expression of an image to provide the adapted image comprising utilization of the information relating to the patient's viewing of one or more images of the plurality of images.

Fig. 2 shows a schematic example of an imaging system 100. The system comprises a magnetic resonance imaging (MRI) scanner 110, and an apparatus 10 for determining and/or assessing depression severity of a patient as described with respect to Fig. 1.

Fig. 3 shows a method 200 for determining and/or assessing depression severity of a patient in its basic steps. The method comprises:

- in a presenting step 210, also referred to as step a), presenting on an image display unit a plurality of images to a patient, and wherein the plurality of images are of different human facial expressions;
- in an acquiring step 220, also referred to as step b), acquiring by at least one sensor information relating to the patient's viewing of the plurality of images;
- in a providing step 230, also referred to as step c), providing by an input unit a processing unit with the information relating to the patient's viewing of the plurality of images;
- in a providing step 240, also referred to as step d), providing by the input unit the processing unit with MRI scan image data of the patient, wherein the MRI scan image data of the patient comprises MRI scan image data of at least one part of the patient's brain associated with recognition of facial expressions and/or associated with affective disorders; and
- in a determining step 250, also referred to as step e), determining and/or assessing by the processing unit depression severity of the patient comprising utilizing the information relating to the patient's viewing of the plurality of images and the MRI image data of at least one part of the patient's brain associated with recognition of facial expressions and/or associated with affective disorders.

It is to be noted that some method steps can be carried out in different orders or at the same time. For example steps a), b) and c) can be carried out, and then step e) can be partially carried out. Then step d) can be carried out followed by a completion of step e). For example, steps a)-b)-c)-d) can be carried out simultaneously followed by step e). For example first parts of steps a)-b) can be carried out before step d) and step c) can be carried out after step d) or step c) can be carried out partially before and after step d), followed by step e). For example step d) can be carried out followed by steps a)-b)-c), followed by step e). For example, step d) can be carried out followed by step e) being partially carried out, followed by steps a)-b)-c), and the step e) completed.

In an example, method comprises presenting by the image display unit the plurality of images to the patient undergoing the MRI scan.

In an example, the method comprises determining by a sensor of the at least one sensor what image of the plurality of images the patient is viewing.

In an example, the method comprises acquiring at least some of the MRI scan image data of the patient during a period when the plurality of images are presented to the patient.

In an example, the method comprises acquiring at least some of the information relating to the patient's viewing of the plurality of images at the same time as the MRI scan image data of at least one part of the patient's brain associated with recognition of facial expressions and/or associated with affective disorders.

In an example, the method comprises acquiring at least some of the information relating to the patient's viewing of the plurality of images at a different time to the MRI scan image data of at least one part of the patient's brain associated with recognition of facial expressions and/or associated with affective disorders

In an example, method comprises presenting different images to the patient in response to input provided by the patient via an interaction input unit of the image display unit, wherein a sensor of the at least one sensor determines what image of the plurality of images the patient is viewing, and wherein the information relating to the patient's viewing of the plurality of images relates to the viewing of one or more images of the plurality of images.

In an example, the method comprises browsing through the plurality of images from one type of emotion or feeling or mood state to another, for example from sad to happy and from happy to sad, via a slidable scale input or push button or touchpad or mouse of the interaction input unit.

In an example, the information relating to the patient's viewing of the plurality of images comprises information relating to an image that the patient views that the patient indicates to equate with a current state of the patient.

In an example, the information relating to the patient's viewing of the plurality of images comprises information relating to at least one image browsing pattern of the patient.

In an example, the information relating to the at least one browsing pattern comprises of one more of: durations that the patient has viewed two or more images of the plurality of images; changes in put by the patient to the interaction input unit with respect to changes in a viewing direction of the plurality of images

In an example, the information relating to the patient's viewing of the plurality of images comprises information relating to one or more specific images viewed by the patient.

In an example, the method comprises tracking the patient's eyes with an eye tracking sensor, and the information relating to the patient's viewing of the plurality of images comprises information relating to where the patient is looking.

In an example, the method comprises utilizing by the processing unit the information relating to the patient's viewing of the plurality of images and the MRI scan image data of at least one part of the patient's brain associated with recognition of facial expressions and/or associated with affective disorders to determine a distribution of assessments of facial expressions for the plurality of images and the determining and/or assessing the depression severity of the patient comprises determining a skewness metric associated with the distribution of assessments of facial expressions with respect to that exhibited by one or more non-depressed patients.

In an example, the method comprises presenting by the image display unit a first image of the plurality of images to the left visual field of the patient and presenting a second image of the plurality of images to the right visual field of the patient, and determining by the processing unit which image of the first image or second image is gaining the most attention from the patient, and the information relating to the patient's viewing of the plurality of images comprises information relating to the image that is gaining the most attention by the patient.

In an example, the MRI image data of the patient comprises image data of at least one part of the brain associated with viewing an image with the right visual field and image data of at least one part of the brain associated with viewing an image with the left visual field, and the method comprises analysing by the processing unit the image data of the at least one part of the brain associated with viewing an image with the right visual field and the image data of at least one part of the brain associated with viewing an image with the left visual field to determine which image is gaining the most attention by the patient.

In an example, an image of the plurality of images is an adapted image, and wherein the method comprises adapting by the processing unit a facial expression of an image to provide the adapted image comprising utilizing the information relating to the patient's viewing of one or more images of the plurality of images.

The inventors realised that the knowledge that patients who suffer, or have suffered from depression, often exhibit bias in their recognition of facial expressions, e.g. they interpret mildly sad expressions as more severe than normal people would do, can be utilized in conjunction with MRI measurements of brain areas involved in processing of facial expressions in order to enable depression severity of the patient to be determined and/or assessed. Thus a new approach, described here, was developed to support the diagnosis and severity assessment of depression, as well as its longitudinal monitoring. In this new approach Magnetic Resonance Imaging (MRI) is used together with the bilateral presentation of images of different facial expressions, or the measurement of gaze with an eye tracker. The MRI measurements can be optimized on the basis of the patient's responses to a facial-expression-based application that allows the patient to register his mood on a daily or even hourly basis, and/or the facial based information can be optimized based on the MRI measurements and/or the facial based information and MRI measurements can be acquired simultaneously and complement one another. This novel approach allows an objective quantification of depression state and severity.

To aid in the understanding of the developments made by the inventors, below there is an outline of the inventive trip undertaken by the inventors, including the research and assessment of literature in this area, which led to the new technological approach.

The inventors established that bias in perception of facial expressions plays a key role in mood disorders: Mood disorders have been associated with disturbances, often called bias, in the processing and perception of facial expressions. (See for example: H. Davies, I. Wolz, J. Leppanen, F. Fernandez-Aranda, U. Schmidt, and K. Tchanturia, "Facial expression to emotional stimuli in non-psychotic disorders: A systematic review and meta-analysis," Neurosci. Biobehav. Rev., vol. 64, pp. 252-271, 2016). Research in various fields of literature has identified that specific disorders influence the way facial expressions are perceived and in turn - interpreted. Moreover, there has found to be a distinct link between particular mood disorders, and a bias in the perception of particular facial expressions, and the inventors realised they could utilize this. The inventors appreciated that this bias depends on a particular mood disorder, and an increasing number of mood disturbances can therefore be associated with activity in a particular brain region, which can be assessed with Magnetic Resonance Imaging (MRI) brain scanning.

Below is an outline of what the current insights on bias imply for facial expression-based assessment interfaces in the domain of psychotherapy: what they need to contain, the kind of data that can be collected and how it can be interpreted. Finally, a conclusion is presented with some insights as to how such a facial expression-based assessment method can improve the status-quo of assessing mood disorders, and how this can be linked to MRI assessments.

From literature, the inventors identified three biases that characterize facial expression processing in mood disorders. These are:

- **attentional bias** in selectively attending to or dwelling on particular facial expressions,
- **temporal bias** expressed as a latency in classifying facial expressions and
- **perceptual bias** represented as misattributing categorical labels to facial expressions.

The literature revealed that particular mood disorders have characteristic biases related to distinct facial expressions. Most works assess the presence of those biases in depression and anxiety.

### Major Depressive Disorder (MDD)

**Attentional bias:** For major depressive disorder (MDD) the inventors established that there are indications that depressed patients tend to direct their gaze towards sad faces and appear to dwell longer on those. This effect is classified as an **attentional bias.** It is not conclusive whether this increased dwell time is attributed to processing needs for sad facial expressions or to a different reason, but it is manifested condition in at least a portion of population suffering from MDD. See for example:

L. Amit, B.-Z. Ziv, S. Dana, P. Daniel S, and B.-H. Yair, "Free viewing of sad and happy faces in depression: A potential target for attention bias modification," Physiol. Behav., vol. 176, no. 3, pp. 139-148, 2017.
L. Leyman, R. De Raedt, R. Schacht, and E. H. W. Koster, "Attentional biases for angry faces in unipolar depression," Psychol. Med., vol. 37, no. 3, pp. 393-402, 2007.
I. H. Gotlib and E. Krasnoperova, "Attentional Biases for Negative Interpersonal Stimuli in Clinical Depression Ian," J. Abnorm. Psychol., vol. 113, no. 1, pp. 127-135, 2004.
A. Duque and C. Vázquez, "Double attention bias for positive and negative emotional faces in clinical depression: Evidence from an eye-tracking study," J. Behav. Ther. Exp. Psychiatry, vol. 46, pp. 107-114, 2015.
J. Joormann and I. H. Gotlib, "Selective attention to emotional faces following recovery from depression," J. Abnorm. Psychol., vol. 116, no. 1, pp. 80-85, 2007

**Temporal bias: Temporal bias** is identified to be present mostly in clinical, subclinical and patients in remission, where there is a slowness in responding to positive faces. Additionally, for depressed patients there is evidence that they recognize neutral expressions more slowly as well. See for example:

J. M. Leppanen, M. Milders, J. S. Bell, E. Terriere, and J. K. Hietanen, "Depression biases the recognition of emotionally neutral faces," Psychiatry Res., vol. 128, no. 2, pp. 123-133, 2004.
Q. Dai and Z. Feng, "More excited for negative facial expressions in depression: Evidence from an event-related potential study," Clin. Neurophysiol., vol. 123, no. 11, pp. 2172-2179, 2012.
S. A. Langenecker, L. A. Bieliauskas, L. J. Rapport, J.-K. Zubieta, E. A. Wilde, and S. Berent, "Face emotion perception and executive functioning deficits in depression.," J. Clin. Exp. Neuropsychol., vol. 27, no. 3, pp. 320-33, 2005

**Perceptual bias**: The **perceptual bias** is defined by a skewed perception of particular or multiple facial expressions. See for example the references detailed above for Temporal bias and also:

M. N. Dalili, I. S. Penton-Voak, C. J. Harmer, and M. R. Munafò, "Meta-analysis of emotion recognition deficits in major depressive disorder," Psychol. Med., vol. 45, no. 6, pp. 1135-1144, 2015.
P. Ekman, "Universal Facial Expressions of Emotion," California Mental Health, vol. 8 (4), no. 4. pp. 151-158, 1970.
J. Joormann and I. H. Gotlib, "Is this happiness i see? Biases in the identification of emotional facial expressions in depression and social phobia," J. Abnorm. Psychol., vol. 115, no. 4, pp. 705-714, 2006.
E. S. Mikhailova, T. V. Vladimirova, A. F. Iznak, E. J. Tsusulkovskaya, and N. V. Sushko, "Abnormal recognition of facial expression of emotions in depressed patients with major depression disorder and schizotypal personality disorder," Biol. Psychiatry, vol. 40, no. 8, pp. 697-705, 1996.
J. K. Gollan, M. McCloskey, D. Hoxha, and E. F. Coccaro, "How do depressed and healthy adults interpret nuanced facial expressions?," J. Abnorm. Psychol., vol. 119, no. 4, pp. 804-810, 2010.
S. A. Surguladze, C. Senior, A. W. Young, G. Brébion, M. J. Travis, and M. L. Phillips, "Recognition Accuracy and Response Bias to Happy and Sad Facial Expressions in Patients with Major Depression," Neuropsychology, vol. 18, no. 2, pp. 212-218, 2004. E. Dejonckheere et al., "The bipolarity of affect and depressive symptoms," J. Pers. Soc. Psychol., vol. 114, no. 2, pp. 323-341, 2018

The inventors realised from the literature that clinical depression does not consistently affect the recognition of happy and sad faces as much as it affects the recognition of neutral faces and expressions with mild intensities. In particular, MDD patients exhibit an impairment in recognizing all mild facial expressions, while retaining an accurate recognition for those of sadness for all intensities. On the other hand, people in remission or those with a sub-clinical form of depression have an impaired perception of mildly happy facial expressions only. Another observation the inventors established is that neutral facial expressions are more frequently misinterpreted by depressed patients. Generally, the recognition accuracy improves with an increase of emotion in the portrayed facial expressions. In sum, the inventors realised that processing of facial emotions, as quantified by psychophysics using eye-tracking (attentional bias), response times (temporal bias) and response accuracy (perceptual bias), can be used to estimate several biases that quantify the severity of a depression.

Thus, the inventors realised that the facial-expression-biases can be combined and enhanced with MR brain imaging in order to determine/assess depression in patients in a new way. The new approach developed by the inventors looked to new imaging approaches that are able to shed light on whether emotions are represented in discernable areas of the brain. The work by Vytal et al. was referred to by the inventors in developing the new technique, with this work collating more than a decade of existing literature indicating that basic emotions are indeed reflected in neural correlates within the brain. See: K. Vytal and S. Hamann, "Neuroimaging support for discrete neural correlates of basic emotions: A voxel-based meta-analysis," J. Cogn. Neurosci., vol. 22, no. 12, pp. 2864-2885, 2010.

Thus, the inventors developed a new approach involving the integration of bias in facial expression and brain imaging to yield new therapeutic solutions and improve health outcomes for a population, which is at the moment restricted in their choice to pharmaceutical or therapy-based solutions. The novel integrative approach can be utilized first as a monitoring tool, to assess the severity of the mental disorder. Then, it can be used as a screening tool to identify areas in the brain, which are correlated to individual basic emotions for which a patient exhibits a perceptual perturbation. Based on evidence from the literature (see for example Vytal et al), the inventors established that regions which are neural correlations of those basic emotions can be precisely located using MRI scans, thus reducing significantly data analysis. As a final step, the inventors realised that new products can be developed and customized, which employ a combination of MR brain imaging and transcranial magnetic stimulation (TMS) specifically tuned to disorder or symptom specificity. The new developments would significantly reduce the time required to develop and personalize such tools.

The developed approach is now discussed in further specific detail with respect to several exemplar embodiments.

Thus, as discussed above a novel diagnostic approach has been developed to support the assessment of depression severity. An intuitive (face-based) user-interface is deployed that includes perception and manipulation of facial expression. This interface allows for face-based mood self-report (see embodiment 1#). This assessment does not rely on extensive verbal or written communication and can be administered multiple times without loss of accuracy. The interface is designed for ecological momentary assessments (EMA), also called experience sampling. Therefore, the assessment is user-friendly and minimally intrusive. The user-interface allows for fast and accurate quantification of the 3-types of biases (perceptual, temporal and recognition biases), so called "bias extraction". When combined with (functional) magnetic resonance imaging (fMRI), these measurements can enable the diagnosis of depression severity.

Knowledge on biases in the perception of facial expressions has been deployed by algorithmically estimating the overall severity of the mood disorder for a particular time period. This is done by analyzing the information obtained from a patient's MRI scans in combination with the below described face-based mood self-reports and extracting the biases in the perception of facial expressions, which are known to be a marker of various states and types of mood disorders.

The bias extraction can be done unobtrusively without any additional user input. An interface is utilized, which uses realistic facial expressions as an input device for providing those self-reports.

In summary before going into specific further details, the main elements of the new development are:
One or more units that are MRI compatible or a new MRI unit, for objective longitudinal assessment: The compatible/modified MRI scanner contains:

Eye tracking unit/system
Unit for presenting facial expressions within the MRI scanner (potentially while registering eye tracking parameters)
A face-based user interface, allowing the assessment of mood using realistic facial expressions as an input for self-reports
Processing and analysis unit:
To analyze the biases present in the facial expressions selected by a particular user in the course of the time period to be assessed.

To link the biases identified to potential types and states of mood disorder.

Below specific embodiments are new discussed.

## Embodiment 1: User-interface data quantifies mood disorders in real life

It is possible to determine visual biases related to mood disorders *before* the patient enters the MRI scanner - but this can also be done during the scan. This can be for instance with a face-based user interface of which the user can change the facial expression by means of a (2 dimensional) slider: for each coordinate of the slider a different facial expression is generated and presented to the user. When the user has browsed this 2-dimensional space and arrived at the facial expression that he thinks represents his current mood best, he selects this facial expression. The facial expression selected thus serves as an indication of the mood of the patient at that moment in time. Since this interface can be presented in a mobile phone application, it can be used longitudinally to compile a detailed pattern of self-reported moods and mood changes over time.

But there is also the possibility to extract additional information about visual biases, namely from the 2-dimensional browse patterns that the user followed before the actual selection: on which facial expressions did he dwell longer? Did the user select relatively less mild sad faces than mild happy faces? Which biases are present? Imaging techniques can then serve to give a confirmation of the observations suggested by the biases found with user-interface based approach. This can be accomplished by administering facial expression-based tests, which will prompt the respective areas of the brain to activate.

The neural activity monitored by the fMRI scanner forms the basis for improved accuracy of the assessment.

In addition, this user-interface derived bias information can be a preparation for the procedures presented in embodiments 1 and 2 above: knowing the biases present in this patient in real life allows a focus of the MRI scans to the brain areas of interest.

In the subsections below it is detailed how exactly the mood-disorder related biases can be determined from the facial-expression interface browse patterns.

## Quantifying the attentional and temporal biases

In a facial expression-based assessment interface, browsing behavior can be represented as a trace of the facial expressions viewed by the user in a single assessment. The trace is defined as an ordered collection of all coordinates of the 2-dimensional slider and the facial expressions they represent, which have been explored by a user in a single assessment. This parameter becomes particularly informative, when each visited coordinate is paired with its respective timestamp. This operationalizes the attentional and temporal biases defined in the background section. Even though the underlying mechanism for why the attentional and temporal biases are expressed is different, an increased latency for happy and sad facial expressions relative to other can be expected, with respect to unaffected facial expressions. Calculating the time required to provide an assessment can be done simply as follows:

$$T = timestamp_{end} - timestamp_{begin},$$

where *timestamp$_{begin}$* can either be the timestamp of the first selection made by a user or the time when the interface was presented, which will also account for the duration in which a person would deliberate prior to making an assessment.

Furthermore, the browsing behavior can also be analyzed for 'oscillations' in the choosing pattern of an accurate facial expression for an assessment. Those are defined as the turning points in the sequence of values for a single assessment. The average number of oscillations can be a marker for sensitivity to certain emotions, which is expressed as specificity in the choice of facial expression intensities for an assessment. Oscillations can be calculated by taking the assessment trace and identifying the turning points as follows:

$$x_{\{i-1\}} < x_{\{i\}} > x_{\{i+1\}} \text{ or } x_{\{i-1\}} > x_{\{i\}} < x_{\{i+1\}}$$

where x is the numerical value for the intensity of the selected facial expression, *i* is the index or order number of that element in the trace and *n* represents all visited coordinates, whose values are indexed as $x_{\{1..n\}}$.

Regarding this "intensity", in an arranged sequence of images depicting a gradient of facial expression intensities ranging from the neutral to a target facial expression, x would refer to the normalized ordinal value of the selected image. For example, 0 would correspond to the neutral expression, 1 - to the target expression at maximum intensity. Provided there are 100 images, 0.5 intensity would be the 50th image.

For single assessments, this information might not be informative, but an aggregation of those characteristics for a number of assessments provides insights into the overall state of a persons' well-being. For example, based on the literature, it was established by the inventors that one can take the relative speed of assessments for particular facial expression over time as a marker of the mood disorders' severity. For people with depression, slower assessment times are expected when using sad and happy facial expressions, as they would be an indicator for both dwelling over sad facial expressions and slower response to happy ones.

**Quantifying the perceptual bias**

The relationship between disorder severity and a lowered perceptual accuracy in the recognition of mild facial expressions can be captured by using the distribution of assessments for the particular facial expression. From the literature, it was established by the inventors that people living with depression show a decreased overall sensitivity in recognizing mild facial expressions with the exception of expressions portraying sadness. Thus the inventors hypothesized that the distribution for those expressions would be negatively skewed to resemble the bias away from mild facial expressions (see Equation 4), and this indeed found functionality in the newly developed approach.

To put it simply, the inventors established that subtle facial expressions would not carry any meaning for depressed patients, which will be reflected in the absence of subtle expression assessments. People in remission and mildly depressed exhibit this bias only for happy facial expressions, while non-depressed people exhibit no such bias. For all groups there is little evidence for decreased perceptual accuracy to sad facial expressions. As such, those will serve as ground truth for an undisturbed perception of mild facial expressions. As such the following use cases under Equation (1), (2) and (3) can be defined, where the parameter P equates to the skewness of the distribution of assessments provided with the respective facial expressions (see Equation 4 and 5), and where specifically P is the perceptual distance or score . Furthermore, the *all* notation refers to all facial expressions excluding the one for sadness.

$$(1) \quad P_{sad} > P_{all}$$

$$(2) \quad P_{sad} > P_{happy} \quad AND \quad P_{sad} \cong P_{all-happy}$$

$$(3) \quad P_{sad} \cong P_{all}$$

1. Depression
2. Remission / Mild depression
3. Healthy

**Equation (1), (2), (3)**

$$P = 50 \quad \text{normal distribution}$$
$$P < 50 \quad \text{negatively skewed}$$
$$P > 50 \quad \text{positively skewed}$$

*Equation 4. Distribution skewness parameters*

$$P = \frac{\mu_d - \nu_d}{\sigma_d}$$

*Equation 5. Skewness formula*

**Quantifying a specific period in time**

Consider an interface, which features a sequence of images for each facial expression spread in the interval [0, 1], then in this case, zero will correspond to the neutral expression and one -- the respective facial expressions' maximum intensity. In order to obtain a quantification of the perceptual bias, the skewness of the distribution for all assessments within a desired time period is calculated. The skewness of the distribution for assessments provided by each facial expression can be calculated. The parameter to be obtained is then calculated by simply subtracting the skewness of the target facial expression from the skewness of the distribution from the sad facial expression as follows:

$$P = S_{all} - S_{sad},$$

where P is the parameter we obtain, which quantifies this perceptual distance, and where S is the skewness.
P should yield a positive value when there are symptoms of depression, disorder severity is positively correlated to P. It is to be noted that for longer periods of time, an overall estimate of the conditions' severity can be derived, but it can be difficult to pinpoint specific periods, where the severity has decreased or increased relative to adjacent periods without explicitly running the algorithm for that particular subset of time.

For the above formulas, the following information is detailed.
P is the perceptual distance or score.

$$Sd = (\mu d - \nu\, d) / \sigma d \quad (1)$$

where:

$S$ = Pearson's Mode Skewness
$\mu$ = Mean
$v$ = Mode
$\sigma$ = Standard Deviation
$d$ = Index denoting particular facial expression

**Monitoring scenario**

Provided there is sufficient and consistent historical monitoring data, the severity of the disorder can also be monitored over time. In order to do that a sliding window approach is used to aggregate the data into points which describe the disorders' severity for a constant retrospective period. In order to do that two parameters are defined. The first parameter is the size of the window, which corresponds to the length of time for historical data we will be taking into account. The second parameter is the step parameter, which will define how much the window for subsequent assessments is moved. To illustrate this with an example, a period of two weeks for the window size and one day for the step parameter can be utilized. This will give a daily estimate of the symptom's severity. Another example could be a window-size of one month with a step parameter of one month as well. This would give an estimate for the disorders' severity on a monthly basis. The benefit of doing this is that it would allow us to be able to visualize the relative change of the disorders' severity over time, but does not alter the way we calculate the parameters

**fMRI**

*Functionality check*

In order to check whether the above-mentioned biases are not affected by dysfunctional neurological processes, real-time fMRI analyses of relevant areas are conducted.

Using real-time fMRI, brain activity from the set of brain regions involved in facial processing can be identified, while the patient uses the face-based user interface. Using a contrast condition, one can measure the activity of the core facial processing network, consisting of the primary visual cortex (VI), the occipital face area (OFA), the fusiform face area (FFA, and the posterior superior temporal sulcus (sPTS) (REF, Kanwisher, Haxby) Together, these connected brain regions are vital for perception of faces. Using a reference group, collected in non-depressed controls, it can be identified if patients show dysfunctional neurological processes in perception. This analysis is a functional check, to clarify if other, for example neurodegeneration or a stroke give rise to a temporal or attentional bias. Furthermore, abnormal activity in the facial processing network can also help identify (or screen) patients with mild forms of prosopagnosia that might have been using the interface. In addition, in order to check whether the facial expressions are actually processed, a real-time and event-related scan of the emotional system (in particular amygdala) will be conducted. This could be used to include/exclude patient interaction with the user-interface and more reliably quantify the attentional, temporal and perceptual bias.

The core facial processing network is also connected to an extended set of brain regions that include amygdala, inferior frontal gyrus (IFG) and precuneus. Using clustering methods, the extended facial processing network can be divided in functional subsystems that are more closely associated with perception (V1, OFA, FFA), semantic association (sPTS, precuneus) and emotional expressions (amygdala, IFG). See for example: [https://www.ncbi.nlm.nih.gov/pmc/articles/PMC3603994/pdf/pone.0059886.pdf}. In addition, to levels of brain activity, information about the effective connectivity between the core (and extended) regions of the facial processing network can be extracted. Abnormal activity, during perception of emotional faces, has been shown to track depression severity, for example in relation to activity in the amydala. Furthermore, measure of effective connectivity, have recently been shown to provide some prognostic value for the clinical trajectory of a depression. See for example; [https:/www.sciencedirect.com/science/article/pii/S2213158220300504]. By real-time analysis of the regions involved in the facial processing network, including methods of effective connectivity, between regions in the core, these measurements can also provide information on the depression severity. Distinct components of the extended facial processing network can further be linked to the distinct biases. For the attentional bias, the connection between attentional networks and the facial processing network are likely important. For the temporal bias, the connectivity with the psychomotor network can be scanned. For the perceptual bias, the connectivity within the extended facial processing network was found to be of relevance.

*Real-time adaptation of stimuli*

A second way of using fMRI in the assessment of depression severity using the facial-expression interface, is that the type of facial expression can be adapted according to the monitored emotional activity of the patient that is being scanned in order to increase the accuracy of the assessment. This can be done by adjustment of the facial expression based on the real-time monitoring of brain activity in a particular area (e.g. amygdala) during a functional MRI scan. So, there is a closed-loop system where the results from the fMRI scan are directly (real-time) fed back to the system to adapt the facial expression.

**Embodiment 2: MR compatible eve tracking system integrated in head coil**

In this embodiment eye tracking measurements are used together with the real-time measured brain activity for the diagnostic purpose of assessing depression severity. For example an infrared eye tracking system is integrated in head coil behind the mirror. The mirror is dielectric and reflects visible light (e.g. the facial expression stimuli from the monitor outside the bore) but transmits invisible infrared light from the infrared camera behind the mirror. See for example the eye tracking system by Cambridge Research Systems (https://www.crsltd.com/tools-for-functional-imaging/mr-safe-eve-tracking-for-fmri/livetrack-fmri/)

Therefore, an MR compatible/modified system is used where eye-tracked device/system is mounted. Additionally, the MR compatible system contains a unit that presents visual information such as pictures or a series of pictures or video material. Upon presentation of the material to the user, an MR compatible eye tracking system will follow the gaze of the user. In a preferred embodiment, the gaze tracker is integrated in the head coil or in the bore of the MR scanner (e.g. a system from Cambridge Research Systems or Philips VitalEye). However, it is to be noted that the eye-tracking device can be used outside of the MRI, before an MRI scan to acquire information as the patient views images, and can then also be used as the patients views these images and/or other images during an MRI scan.

During the eye tracking measurements several different parameters can be assessed: fixations and gaze points, heat maps, areas of interest, time to first fixation, total time spend, ratio, fixation sequences, revisits, first fixation duration, average fixation duration, pupil dilation. The sensorial information may be presented consecutively or simultaneously at different spatial positions presented during the time that patient is lying in the MR scanner.

Note that for this particular purpose, material is presented with positive valence (e.g. happy faces) of variable intensity (from a mildly content facial expression towards an euphoric facial expression) as well as material with negative valence (e.g. sad faces) of variable intensity. Visual presentation of facial expressions can be combined with e.g. fragrance of different valence (e.g. a stinky fragrance or a comforting fragrance). Note that adding sensory modalities increases the intensity of the experience.

The depression severity can be assessed on a continuous scale with numeric dependent variables as the examples from the eye tracker specified above. The diagnostic severity is quantified through the differential parameter values obtained by the eye tracking for negatively valanced stimuli (facial expressions) versus positively valanced stimuli. It is known that patients suffering from mental health problems pay more attention to negatively valanced stimuli. This attention can be numerically quantified through the eye gaze tracking parameters. This information is ideally combined with the real-time measured activation of the amygdala. It is seen in patients suffering from depression that also at the neural level, they show an increased bilateral response to the negative faces in the limbic system. Since the information obtained by means of eye tracking is combined together with neural information collected while presenting multisensorial signals, the new approach provides a more reliable information of depression severity.

**Embodiment 3: Retinotopical VI quantifies visual attention biases**

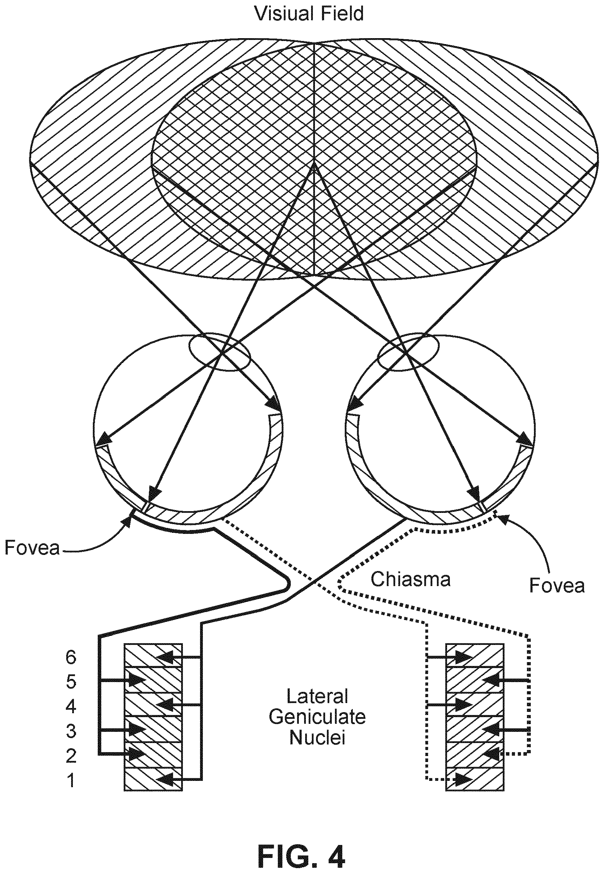

The visual system is retinotopically organized, as shown in Fig. 4 and the inventors realised that they could make use of this in a new way for the determination/assessment of depressive severity. This means that the position on the retina determines which cells in the calcarine cortex will activate. The left visual field will project in the right visual cortex, whilst the right visual field will project in the left visual cortex (contralateral activation). The top visual field will project onto the bottom visual cortex and the bottom visual field will project onto the top visual cortex.

In this embodiment use can be made of this spatial organization of the visual system to replace the eye tracking system. A facial "task" can be designed in a way to project two facial expressions at the same time but one in the left visual field and one in the right visual field. Then it can be determined from the activation in the calcarine cortex how much time is being attended to either visual field. The two facial expressions should be one with positive valence and one with negative valence, such that it can be measured to which valence most attention goes. It was established by the inventors from the literature that patients suffering from depression pay most attention towards the negatively valanced stimuli. With this measurement technique, it can be exactly specified how strong the attention bias is, and consequently this information can be used for the diagnosis of the illness severity. Next to these calcarine cortex activation parameters, the activation of the limbic system can also be incorporated in the bias calculation because this enables to determine whether the facial expression has increased the emotional intensity. Taken together, the information coming from these two activation centers is used to determine illness severity.

**Embodiment 4: use face of one self**

In a further embodiment the face presentation is more specific and more individualized: The method from embodiment 1 is employed using a picture (or a rendering) of the patient's own face.

In another exemplary embodiment, a computer program or computer program element is provided that is characterized by being configured to execute the method steps of the method according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. An apparatus (10) for determining and/or assessing depression severity of a patient, the apparatus comprising:

   - an input unit (20);
   - an image display unit (30);
   - at least one sensor (40); and
   - a processing unit (50);

   wherein, the image display unit is configured to present a plurality of images to a patient and wherein the plurality of images are of different human facial expressions;
   wherein, the at least one sensor is configured to acquire information relating to the patient's viewing of the plurality of images;
   wherein, the input unit is configured to provide the processing unit with the information relating to the patient's viewing of the plurality of images;
   wherein, the input unit is configured to provide the processing unit with MRI scan image data of the patient, wherein the MRI scan image data of the patient comprises MRI scan image data of at least one part of the patient's brain associated with recognition of facial expressions and/or associated with affective disorders; and
   wherein, the processing unit is configured to determine and/or assess depression severity of the patient comprising utilization of the information relating to the patient's viewing of the plurality of images and the MRI scan image data of at least one part of the patient's brain associated with recognition of facial expressions and/or associated with affective disorders.

2. Apparatus according to claim 1, wherein at least some of the MRI scan image data of the patient comprises MRI scan image data of the patient acquired during a period when the plurality of images were presented to the patient.

3. Apparatus according to any of claims 1-2, wherein at least some of the information relating to the patient's viewing of the plurality of images was acquired at the same time as the MRI scan image data of at least one part of the patient's brain associated with recognition of facial expressions and/or associated with affective disorders.

4. Apparatus according to any of claims 1-3, wherein the image display unit comprises an interaction input unit configured to present different images to the patient in response to input provided by the patient, wherein a sensor of the at least one sensor is configured to determine what image of the plurality of images the patient is viewing, and wherein the information relating to the patient's viewing of the plurality of images relates to the viewing of one or more images of the plurality of images.

5. Apparatus according to any of claims 1-4, wherein the information relating to the patient's viewing of the plurality of images comprises information relating to at least one image browsing pattern of the patient.

6. Apparatus according to claim 5, wherein the information relating to the at least one browsing pattern comprises of one more of: durations that the patient has viewed two or more images of the plurality of images; changes in input by the patient to the interaction input unit with respect to changes in a viewing direction of the plurality of images.

7. Apparatus according to any of claims 1-6, wherein a sensor of the at least one sensor comprises an eye tracking sensor, and wherein the information relating to the patient's viewing of the plurality of images comprises information relating to where the patient is looking.

8. Apparatus according to any of claims 1-7, wherein the processing unit is configured to utilize the information relating to the patient's viewing of the plurality of images and the MRI scan image data of at least one part of the patient's brain associated with recognition of facial expressions and/or associated with affective disorders to determine a distribution of assessments of facial expressions for the plurality of images and wherein the determination and/or assessment of the depression severity of the patient comprises a determination of a skewness metric associated with the distribution of assessments of facial expressions with respect to that exhibited by one or more non-depressed patients.

9. Apparatus according to claim 1, wherein the image display unit is configured to present a first image of the plurality of images to the left visual field of the patient and present a second image of the plurality of images to the right visual field of the patient, wherein the processing unit is configured to determine which image of the first image or second image is gaining the most attention from the patient, and wherein information relating to the patient's viewing of the plurality of images comprises information relating to the image that is gaining the most attention by the patient.

10. Apparatus according to claim 9, wherein the MRI image data of the patient comprises image data of at least one part of the brain associated with viewing an image with the right visual field and image data of at least one part of the brain associated with viewing an image with the left visual field, and wherein the processing unit is configured to analyse the image data of the at least one part of the brain associated with viewing an image with the right visual field and the image data of at least one part of the brain associated with viewing an image with the left visual field to determine which image is gaining the most attention by the patient.

11. Apparatus according to any of claims 1-10, wherein an image of the plurality of images is an adapted image, wherein the processing unit is configured to adapt a facial expression of an image to provide the adapted image comprising utilization of the information relating to the patient's viewing of one or more images of the plurality of images.

12. An imaging system (100) comprising;

- a magnetic resonance imaging (MRI) scanner (110); and
- an apparatus (10) for determining and/or assessing depression severity of a patient according to any of claims 1-11.

13. A method (200) for determining and/or assessing depression severity of a patient, the method comprising:

a) presenting (210) on an image display unit a plurality of images to a patient, and wherein the plurality of images are of different human facial expressions;
b) acquiring (220) by at least one sensor information relating to the patient's viewing of the plurality of images;
c) providing (230) by an input unit a processing unit with the information relating to the patient's viewing of the plurality of images;
d) providing (240) by the input unit the processing unit with MRI scan image data of the patient, wherein the MRI scan image data of the patient comprises MRI scan image data of at least one part of the patient's brain associated with recognition of facial expressions and/or associated with affective disorders; and
e) determining and/or assessing (250) by the processing unit depression severity of the patient comprising utilizing the information relating to the patient's viewing of the plurality of images and the MRI image data of at least one part of the patient's brain associated with recognition of facial expressions and/or associated with affective disorders.

14. A computer program element for controlling an apparatus according to one of claims 1 to 11 and/or system of claim 12, which when executed by a processor is configured to carry out the method of claim 13.

15. A computer readable medium having stored the computer program element of claim 14.

10

30

20

50

40

**FIG. 1**

100

110

10

**FIG. 2**

200

a) 210

b) 220

c) 230

d) 240

e) 250

**FIG. 3**

FIG. 4

Europäisches
Patentamt

European
Patent Office

Office européen
des brevets

# EUROPEAN SEARCH REPORT

Application Number

EP 20 20 7161

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FU CYNTHIA H. Y. ET AL: "Attenuation of the Neural Response to Sad Faces in Major Depressionby Antidepressant Treatment : A Prospective, Event-Related Functional Magnetic Resonance ImagingStudy", ARCHIVES OF GENERAL PSYCHIATRY, [Online] vol. 61, no. 9, 1 September 2004 (2004-09-01), pages 877-889, XP055773400, US ISSN: 0003-990X, DOI: 10.1001/archpsyc.61.9.877 | 1-3,8, 11-15 | INV. A61B5/055 A61B5/16 ADD. G01R33/48 |
| Y | * abstract * | 4-7,9 | |
| A | * section METHODS; pages 878-880 * * pages 884-886 * | 10 | |
| Y | DUQUE ALMUDENA ET AL: "Double attention bias for positive and negative emotional faces in clinical depression: Evidence from an eye-tracking study", JOURNAL OF BEHAVIOR THERAPY AND EXPERIMENTAL PSYCHIATRY, vol. 46, 1 March 2015 (2015-03-01), pages 107-114, XP055775304, GB ISSN: 0005-7916, DOI: 10.1016/j.jbtep.2014.09.005 * section 2. Methods; pages 108-110 * | 4-7,9 | |
| X | US 2013/102918 A1 (ETKIN AMIT [US] ET AL) 25 April 2013 (2013-04-25) * paragraphs [0054] - [0058] * * paragraphs [0062] - [0072] * * figures 1-3 * * claims * | 1-3, 12-15 | |

TECHNICAL FIELDS
SEARCHED (IPC)

A61B
G01R

The present search report has been drawn up for all claims

| Place of search | Date of completion of the search | Examiner |
|---|---|---|
| The Hague | 12 February 2021 | Bataille, Frédéric |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 ...................................................................

& : member of the same patent family, corresponding
document

EPO FORM 1503 03.82 (P04C01)

**EP 4 000 511 A1**

## ANNEX TO THE EUROPEAN SEARCH REPORT
## ON EUROPEAN PATENT APPLICATION NO.

EP 20 20 7161

12-02-2021

| Patent document cited in search report | Publication date | Patent family member(s) | Publication date |
|---|---|---|---|
| US 2013102918 A1 | 25-04-2013 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **H. DAVIES ; I. WOLZ ; J. LEPPANEN ; F. FERNANDEZ-ARANDA ; U. SCHMIDT ; K. TCHANTURIA.** Facial expression to emotional stimuli in non-psychotic disorders: A systematic review and meta-analysis. *Neurosci. Biobehav. Rev.,* 2016, vol. 64, 252-271
- **AMIT ; B.-Z. ZIV ; S. DANA ; P. DANIEL S ; B.-H. YAIR.** Free viewing of sad and happy faces in depression: A potential target for attention bias modification. *Physiol. Behav.,* 2017, vol. 176 (3), 139-148
- **LEYMAN ; R. DE RAEDT ; R. SCHACHT ; E. H. W. KOSTER.** Attentional biases for angry faces in unipolar depression. *Psychol. Med.,* 2007, vol. 37 (3), 393-402
- **H. GOTLIB ; E. KRASNOPEROVA.** Attentional Biases for Negative Interpersonal Stimuli in Clinical Depression Ian. *J. Abnorm. Psychol.,* 2004, vol. 113 (1), 127-135
- **A. DUQUE ; C. VÁZQUEZ.** Double attention bias for positive and negative emotional faces in clinical depression: Evidence from an eye-tracking study. *J. Behav. Ther. Exp. Psychiatry,* 2015, vol. 46, 107-114
- **J. JOORMANN ; I. H. GOTLIB.** Selective attention to emotional faces following recovery from depression. *J. Abnorm. Psychol.,* 2007, vol. 116 (1), 80-85
- **J. M. LEPPANEN ; M. MILDERS ; J. S. BELL ; E. TERRIERE ; J. K. HIETANEN.** Depression biases the recognition of emotionally neutral faces. *Psychiatry Res.,* 2004, vol. 128 (2), 123-133
- **Q. DAI ; Z. FENG.** More excited for negative facial expressions in depression: Evidence from an event-related potential study. *Clin. Neurophysiol.,* 2012, vol. 123 (11), 2172-2179

- **S. A. LANGENECKER ; L. A. BIELIAUSKAS ; L. J. RAPPORT ; J.-K. ZUBIETA ; E. A. WILDE ; S. BERENT.** Face emotion perception and executive functioning deficits in depression. *J. Clin. Exp. Neuropsychol.,* 2005, vol. 27 (3), 320-33
- **N. DALILI ; I. S. PENTON-VOAK ; C. J. HARMER ; M. R. MUNAFÒ.** Meta-analysis of emotion recognition deficits in major depressive disorder. *Psychol. Med.,* 2015, vol. 45 (6), 1135-1144
- **P. EKMAN.** Universal Facial Expressions of Emotion. *California Mental Health,* 1970, vol. 8 (4), 151-158
- **J. JOORMANN ; I. H. GOTLIB.** Is this happiness i see? Biases in the identification of emotional facial expressions in depression and social phobia. *J. Abnorm. Psychol.,* 2006, vol. 115 (4), 705-714
- **E. S. MIKHAILOVA ; T. V. VLADIMIROVA ; A. F. IZNAK ; E. J. TSUSULKOVSKAYA ; N. V. SUSHKO.** Abnormal recognition of facial expression of emotions in depressed patients with major depression disorder and schizotypal personality disorder. *Biol. Psychiatry,* 1996, vol. 40 (8), 697-705
- **J. K. GOLLAN ; M. MCCLOSKEY ; D. HOXHA ; E. F. COCCARO.** How do depressed and healthy adults interpret nuanced facial expressions?. *J. Abnorm. Psychol.,* 2010, vol. 119 (4), 804-810
- **S. A. SURGULADZE ; C. SENIOR ; A. W. YOUNG ; G. BRÉBION ; M. J. TRAVIS ; M. L. PHILLIPS.** Recognition Accuracy and Response Bias to Happy and Sad Facial Expressions in Patients with Major Depression. *Neuropsychology,* 2004, vol. 18 (2), 212-218
- **E. DEJONCKHEERE et al.** The bipolarity of affect and depressive symptoms. *J. Pers. Soc. Psychol.,* 2018, vol. 114 (2), 323-341
- **K. VYTAL ; S. HAMANN.** Neuroimaging support for discrete neural correlates of basic emotions: A voxel-based meta-analysis. *J. Cogn. Neurosci.,* 2010, vol. 22 (12), 2864-2885